# EUROPÄISCHE PATENTSCHRIFT

(11) **EP 0 383 128 B1**
(45) Veröffentlichungstag und Bekanntmachung des Hinweises auf die Patenterteilung: **31.03.1993**
(21) Anmeldenummer: 90102178.2
(22) Anmeldetag: 03.02.1990
(51) Int. Cl.: C07C 53/21, C07C 53/50, C07C 69/63, C07C 67/307, C07C 57/54, C07C 51/377, C07C 51/363

(54) **Verfahren zur Fluorierung von Acrylsäure und Derivaten davon sowie neue fluorierte Ester der 2,3-Difluorpropionsäure**
Process for the fluorination of acrylic acid and derivatives thereof, and fluorinated esters of 2,3-difluoropropionic acid
Procédé de fluoration d'acide acrylique et ses dérivés et esters d'acide 2,3-di-fluoropropionique

(30) Priorität: 16.02.1989 DE 3904707
(43) Veröffentlichungstag der Anmeldung: 22.08.1990
(73) Patentinhaber: BAYER AG, 51368 Leverkusen (DE)
(72) Erfinder: Bielefeldt, Dietmar, Dr., D-4030 Ratingen 6 (DE); Gassen, Karl-Rudolf, Dr., D-5068 Odenthal (DE); Lange, Walter, Dr., D-5000 Köln 60 (DE)

(56) Entgegenhaltungen:
- DE-C- 721 180
- GB-A- 730 328
- GB-A- 794 360
- US-A- 2 680 764

## Beschreibung

Die vorliegende Erfindung betrifft ein Verfahren zur Herstellung von Difluorpropionsäure und Derivaten davon durch Fluorierung entsprechender Acrylsäuren bzw. Acrylsäure-Derivaten und neue, auf diese Weise zugängliche fluorierte Ester der 2,3-Difluorpropionsäure.

Polymere α-Fluoracrylsäurederivate besitzen gute optische und mechanische Eigenschaften. Sie sind deshalb zur Herstellung von optischen Vorrichtungen (z.B. Linsen, Compact-Discs) sowie von Fasern und Membranen geeignet (siehe z.B. EP-A1 0 249 867, EP-A2 0 203 462 und US 4 297 466). Bisher haben diese Materialien allerdings noch keine kommerzielle Anwendung gefunden, da kostengünstige und technisch gut handhabbare Verfahren zur Herstellung von α-Fluoracrylsäure und deren Derivaten bislang nicht zur Verfügung stehen. Die bekannten Verfahren erfordern eine Vielzahl von Reaktionsschritten und/oder erbringen die gewünschten Produkte nur in geringen Ausbeuten (siehe z.B. Zh. Org. Khim. 28, 1173 (1987)).

Es wurde nun ein Verfahren zur Fluorierung von Acrylsäure und Derivaten davon gefunden, das dadurch gekennzeichnet ist, daß man an die C=C-Doppelbindung von Acrylsäure oder einem Derivat davon mit der Formel (II) 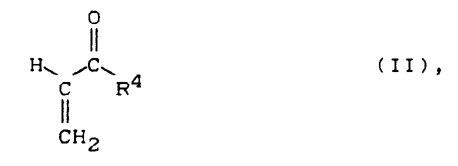
in der
R⁴ für OH, ein Halogenatom oder einen gegebenenfalls substituierten Rest aus der Gruppe C₁- bis C₂₀-Alkoxy, C₃- bis C₂₀-Cycloalkoxy, C₆- bis C₂₀-Aryloxy und C₇- bis C₂₀-Aralkoxy steht
in Gegenwart eines Lösungsmittels elementares Fluor addiert und so 2,3-Difluorpropionsäure oder ein Derivat davon mit der Formel (III) erhält 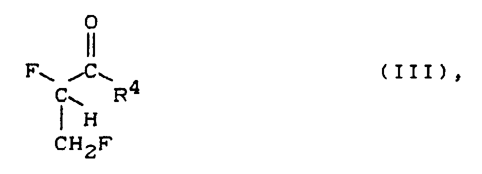
in der
R⁴ die bei Formel (II) angegebene Bedeutung hat.

Durch Abspaltung von Fluorwasserstoff, z.B. durch Zusatz einer Base und gegebenenfalls vor, während und/oder nach der Abspaltung von Fluorwasserstoff vorgenommene Umderivatisierungen, kann man aus Verbindungen der Formel (III) Verbindungen der Formel (I) erhalten 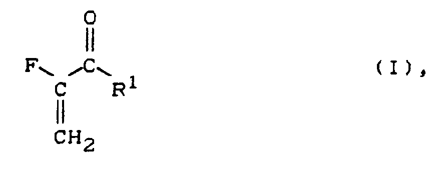
in der
R¹ für NR²R³ oder OR² steht, wobei R² Wasserstoff oder einen gegebenenfalls substituierten Rest aus der Gruppe C₁- bis C₂₀-Alkyl, C₃- bis C₂₀-Cycloalkyl, C₆-bis C₂₀-Aryl und C₇- bis C₂₀-Aralkyl bedeutet und R³ den gleichen Bedeutungsumfang hat wie R² und gleich oder verschieden von R² sein kann.

Soweit in Formel (I) R² und/oder R³ für einen gegebenenfalls substituierten Rest stehen, kommen als Substituenten vorzugsweise C₁- bis C₄-Alkylreste und Halogenatome, insbesondere Fluor- und Chloratome infrage.

Soweit in den erfindungsgemäß einzusetzenden Verbindungen der Formel (II) R⁴ für Halogen steht, handelt es sich um Acrylsäurehalogenide. Bevorzugt sind davon Acrylsäurefluorid und Acrylsäurechlorid. Diese sind gut zugänglich. Besonders bevorzugt ist der Einsatz von Acrylsäurechlorid (Formel (II), R⁴ = Cl).

Soweit in Formel (II) R⁴ für einen gegebenenfalls substituierten Alkoxy-, Cycloalkoxy-, Aryloxy- oder Aralkoxyrest steht sind Halogensubstituenten, insbesondere Fluor und Chlor bevorzugt. Besonders bevorzugt ist Fluor. Bevorzugt sind ferner teilfluorierte aliphatische C₄- bis C₂₀-Alkoxyreste, insbesondere C₄- bis C₇-Alkoxyreste, die geradkettig, verzweigtkettig oder cyclisch sind.

Verbindungen der Formel (II) mit R⁴ = gegebenenfalls mit Halogen substituiertem Alkoxy-, Cycloalkoxy-, Aryloxy-oder Aralkoxyresten sind gut zugänglich (siehe z.B. B. Boutevin, G. Rigal, A. Rousseau; J. Fluorine Chem. 38, 47 (1988)).

Die Umsetzung mit elementarem Fluor ist ein wesentliches Merkmal der vorliegenden Erfindung. Man kann diese Umsetzung beispielsweise bei Temperaturen im Bereich -100 bis +30°C vornehmen. Bevorzugt sind Temperaturen im Bereich -78 bis ±0°C. Vorzugsweise setzt man 0,8 bis 1,5 Mol elementares Fluor pro Mol der Verbindung der Formel (II) ein. Als Lösungsmittel für diese Umsetzung kommen insbesondere fluorierte, gegebenenfalls zusätzlich chlorierte Kohlenwasserstoffe mit Schmelzpunkten unterhalb +20°C in Frage. Ein bevorzugtes Lösungsmittel ist CFCl₃.

Das elementare Fluor kommt im allgemeinen gemischt mit einem Inertgas zum Einsatz. Beispiele für solche Inertgase sind Stickstoff und Helium. Die Fluor/Inertgas-Mischungen können z.B. 0,5 bis 50 Gew.-% Fluor enthalten, bevorzugt sind Gehalte von 1 bis 5 Gew.-% Fluor. Die Umsetzung mit Fluor kann beispielsweise in Gefäßen durchgeführt werden, die aus Polyethylen oder Fluor enthaltenden Polyolefinen, z.B. Polytetrafluorethylen, hergestellt oder damit ausgekleidet sind. Auch Glas, Edelstahl, Nickel, Nickellegierungen, Kupfer und Kupferlegierungen können verwendet werden. Bei einer besonderen Ausführungsform des erfindungsgemäßen Verfahrens wird die Fluorierung in Gegenwart eines HF-Scavengers durchgeführt. Dafür sind z.B. geeignet: Alkali- und Erdalkalifluoride, Alkali- und Erdalkalioxide und Aluminiumoxide. Bevorzugt sind Natriumfluorid und Aluminiumoxid.

Nach der erfindungsgemäßen Umsetzung mit elementarem Fluor erhält man in guten Ausbeuten 2,3-Difluorpropionsäure oder ein Derivat davon (siehe Formel (III)). Unerwünschte Veränderungen am Rest R⁴ und sonstige Nebenreaktionen treten nur in geringem Umfang auf. Die erhaltene Verbindung der Formel (III) kann man beispielsweise isolieren, indem man gegebenenfalls im Reaktionsgemisch vorhandene feste Bestandteile abfiltriert und anschließend das Lösungsmittel abzieht. Gegebenenfalls kann man die Verbindung der Formel (III) durch Destillation weiter reinigen.

Die so erhältlichen Produkte der Formel (III) können in α-Fluoracrylsäurederivate oder in α-Fluoracrylsäure überführt werden. Die Überführung der 2,3-Difluorpropionsäuregruppe in eine α-Fluoracrylsäuregruppe kann mit der Abspaltung von HF durch Zusatz einer Base erreicht werden.

Als Basen kommen hierfür beispielsweise organische Amine, Alkoholate, Alkali- und/oder Erdalkalihydroxide in Frage. Bevorzugt sind tertiäre Amine, wie Dimethylanilin, Triethylamin, 1,5-Diazabicyclo[4.3.0]non-5-en(DBN) und 1,8-Diazabicyclo[5.4.0]undec-7-en (DBU).

Für die Abspaltung von HF wird stöchiometrisch ein Äquivalent Base pro Mol einer Verbindung der Formel (III) oder eines Abwandlungsprodukts davon benötigt. Man setzt deshalb für die Abspaltung von HF im allgemeinen 0,8 bis 1,2 Äquivalente Base ein. In gewissen Fällen ist es angezeigt größere Mengen Base einzusetzen. Wird beispielsweise ein Säurehalogenid, z.B. CH₂F-CHF-CO-Cl, in die HF-Abspaltung bei gleichzeitiger Derivatisierung eingesetzt, so wird ein weiteres Äquivalent Base erforderlich. Wenn man Säureamide herstellen will (siehe Formel (I), R¹ = NR²R³) so ist zur Einführung der NR²R³-Gruppe stöchiometrisch ein Äquivalent des entsprechenden Amins HNR²R³ erforderlich. Dieses Amin kann auch die HF-Eliminierung übernehmen. Dazu ist dann ein weiteres Äquivalent davon erforderlich. Wird für die Einführung einer NR²R³-Gruppe das entsprechende Amin HNR²R³ als Salz, z.B. als Hydrochlorid, eingesetzt, so wird stöchiometrisch ein weiteres Äquivalent Base zur Freisetzung des Amins aus dem Salz benötigt. In allen diesen Fällen ist es angezeigt entsprechend größere Mengen als 0,8 bis 1,2 Äquivalente Base einzusetzen, da die angesprochenen Nebenreaktionen häufig parallel zur HF-Abspaltung ablaufen.

Die Abspaltung von HF kann beispielsweise bei Temperaturen im Bereich von -78 bis +100°C durchgeführt werden. Bevorzugt sind Temperaturen im Bereich von -20 bis +55°C.

Als Lösungsmittel kommen solche in Betracht, die üblicherweise bei der Veresterung oder Amidierung von Carbonyl-Verbindungen verwendet werden. Bevorzugt sind Ether, halogenierte Kohlenwasserstoffe und aromatische Lösungsmittel.

Mit den bisher beschriebenen Maßnahmen kann man Verbindungen der Formel (II) in Verbindungen der Formel (I) überführen, wenn R⁴ in Formel (II) mit R¹ in Formel (I) identisch ist. Beispielsweise kann man so aus einem Acrylsäureester (Formel (II), R⁴ ungleich Halogen und ungleich OH) den analogen α-Fluoracrylsäureester (Formel (I), R¹ = OR², R² ungleich Wasserstoff) herstellen.

Zur Herstellung anderer Verbindungen der Formel (I) können vor, während und/oder nach der HF-Abspaltung Derivatisierungen vorgenommen werden. Diese können auf an sich bekannte Weise durchgeführt werden. Als Beispiele für derartige vor, während und/oder nach der Abspaltung von HF vornehmbare Derivatisierungen seien die folgenden angeführt:
1) 2,3-Difluorpropionsäurehalogenide (Formel (III), R⁴ = Halogen) können durch Reaktion mit einem Alkohol der Formel HOR² (R² ungleich Wasserstoff, sonst wie bei Formel (I) angegeben) in den entsprechenden 2,3-Difluorpropionsäureester umgewandelt werden, aus dem durch HF-Abspaltung Verbindungen der Formel (I) mit R¹ = OR² (R² ungleich Wasserstoff) zugänglich sind. Diese Arbeitsweise ist z.B. vorteilhaft, wenn man Verbindungen der Formel (I) herstellen möchte, die einen schwer zugänglichen und damit teuren Rest OR² enthalten, der bei der Fluorierung zu Nebenreaktionen neigt.
2) α-Fluoracrylsäurehalogenide des Typs CH₂=CF-CO-Hal können durch Reaktion mit einem Alkohol der Formel HOR² (R² ungleich Wasserstoff, sonst wie bei Formel (I) angegeben) in den entsprechenden α-Fluoracrylsäureester (Formel (I), R¹ = OR², R² ungleich Wasserstoff) umgewandelt werden. Diese Arbeitsweise ist z.B. vorteilhaft wenn man Verbindungen der Formel (I) herstellen möchte, die einen schwer zugänglichen und damit teuren Rest OR² enthalten, der bei der HF-Abspaltung zu Nebenreaktionen neigt.
3) 2,3-Difluorpropionsäureester (Formel (III), R⁴ ungleich Halogen, sonst wie bei Formel (II) angegeben) können mit einem Alkohol der Formel HOR² (R² ungleich Wasserstoff, sonst wie bei Formel (I) angegeben) umgeestert und durch anschließende HF-Abspaltung in Verbindungen der Formel (I) mit R¹ = OR² (R² ungleich Wasserstoff) überführt werden. Vor der Umsetzung mit dem Alkohol der Formel HOR² kann der eingesetzte 2,3-Difluorpropionsäureester gegebenenfalls zur freien Säure verseift werden (z.B. mit Salpetersäure) und/oder in das Säurechlorid umgewandelt werden. Auch diese Arbeitsweise ist z.B vorteilhaft, wenn man Verbindungen der Formel (I) herstellen möchte, die einen Rest OR² enthalten, der bei der Fluorierung zu Nebenreaktionen neigt.
4) 2,3-Difluorpropionsäurehalogenide (Formel (III), R⁴ = Halogen) kann man durch Reaktion mit einem Amin der Formel HNR²R³ (R² und R³ wie bei Formel (I) angegeben) in das entsprechende 2,3-Difluorpropionsäureamid überführen, aus dem man durch HF-Abspaltung das entsprechende α-Fluoracrylsäureamid (Formel (I), R¹ = NR²R³) erhalten kann.
5) α-Fluoracrylsäureamide (Formel (I), R¹ = NR²R³) können aus α-Fluoracrylsäurehalogeniden des Typs CH₂ = CF-CO-Hal durch Reaktion mit einem Amin der Formel HNR²R³ erhalten werden.

Auf die beschriebene Weise können Verbindungen der Formel (I) in wenigen Reaktionsschritten, aus gut zugänglichen Ausgangsverbindungen und in guten Ausbeuten erhalten werden. Bevorzugt werden Verbindungen der Formel (I) hergestellt, bei denen R¹ für einen gegebenenfalls substituierten C₂- bis C₁₀-Alkoxyl-, für einen gegebenenfalls substituierten C₃- bis C₈-Cycloalkoxy-, für einen gegebenenfalls substituierten Aryloxy- oder einen gegebenenfalls mit geradkettigen oder cyclischen C₁- bis C₁₀-Alkyl- oder Halogenalkylgruppen substituierten Amidorest steht. Soweit hier substituierte Reste vorliegen handelt es sich vorzugsweise um solche, die mit Halogenatomen, insbesondere Fluor- und/oder Chloratomen substituiert sind.

Die vorliegende Erfindung betrifft weiterhin neue fluorierte Ester der 2,3-Difluorpropionsäure der Formel (III a) 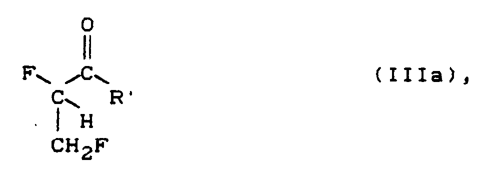
in der
R′ für einen mit Fluor substituierten C₁- bis C₂₀-Alkoxy- oder C₃- bis C₂₀-Cycloalkoxyrest stehen.

An den Alkoxy- und Cycloalkoxyresten können gegebenenfalls außer Fluor weitere Substituenten vorhanden sein, z. B. Chlor.

### Beispiele

### Beispiel 1

In eine Lösung von 560 g (5,6 Mol) Acrylsäureethylester in 7000 ml Trichlorfluormethan wurden bei -78°C 5,6 Mol Fluor (5 %ige Mischung mit Stickstoff, Einleitungsrate 0,5 Mol/Std.) eingeleitet. Das Lösungsmittel wurde danach abdestilliert, und es wurde ein Produktgemisch erhalten, das 31 Gew.-% unumgesetztes Ausgangsprodukt, 6 Gew.-% α-Fluoracrylsäureethylester, 41 Gew.-% 2,3-Difluorpropionsäureethylester und 12 Gew.-% 2,3-Difluorpropionsäuremonofluorethylester enthielt.

### Beispiel 2

In eine Suspension aus 1114 g (12,3 Mol) Acrylsäurechlorid, 615 g (24,6 Mol) Natriumfluorid und 6000 ml Trichlorfluormethan wurden bei -78 bis -65°C 7,5 Mol Fluor (30 %ige Mischung mit Helium, Einleitungsrate 0,5 Mol/Std.) eingeleitet. Anschließend wurde filtriert und aus dem Filtrat 2,3-Difluorpropionsäurechlorid mit einem Siedepunkt von 112 bis 113°C bei 1013 mbar und in einer Reinheit von 98 % durch Destillation gewonnen.

### Beispiel 3

In eine Suspension aus 10 g (0,14 Mol) Acrylsäure, 10 g (0,24 Mol) Natriumfluorid und 100 ml Trichlorfluormethan wurden bei den gleichen Bedingungen wie in Beispiel 2 angegeben 0,15 Mol Fluor eingeleitet. Anschließend wurde filtriert und aus dem Filtrat das Lösungsmittel abgezogen. So wurden 8,5 g 2,3-Difluorpropionsäure in 90 %iger Reinheit erhalten.

### Beispiel 4

2340 g eines wie in Beispiel 1 erhaltenen rohen Fluorierungsgemisches wurden mit 5000 ml 50 gew.-%iger Salpetersäure bis zum Ende der Gasentwicklung bei Raumtemperatur gerührt, was 16 Stunden in Anspruch nahm. Danach wurde das Reaktionsgemisch durch Destillation bei 16 mbar aufgearbeitet. In einer Ausbeute von 394 g wurde 2,3-Difluorpropionsäure mit einem Siedepunkt bei 16 mbar von 82 bis 85°C und einem Schmelzpunkt von 36 bis 38°C erhalten. Das ¹⁹F-Kernresonanzspektrum zeigte charakteristische Linien bei 119 ppm und 152 ppm.

### Beispiel 5

220 g (2,0 Mol) der gemäß Beispiel 4 erhaltenen 2,3-Difluorpropionsäure wurden mit 350 g (2,94 Mol) Thionyl-chlorid bei 90°C gerührt. Als die Gasentwicklung schwächer wurde, wurden 0,5 ml Dimethylformamid zugesetzt und bis zum Ende der Gasentwicklung weitergerührt (insgesamt 18 Stunden). Anschließend wurde das Reaktionsgemisch destilliert. Es wurden 229 g 2,3-Difluorpropionsäurechlorid mit einem Siedepunkt von 112 bis 113°C in einer Reinheit von 98 % erhalten.

### Beispiel 6

Zu einer Lösung von 50 g (0,27 Mol) 2,2,3,4,4,4-Hexafluorbutan-1-ol und 21,6 g (0,27 Mol) Pyridin in 200 ml trockenem Tetrachlormethan wurden 34,6 g (0,27 Mol) 2,3-Difluorpropansäurechlorid getropft. Es wurde 1 Stunde nachgerührt, dann auf Wasser gegossen, extrahiert, getrocknet und destilliert. Auf diese Weise wurden 56 g 2,3-Difluorpropionsäure-2,2,3,4,4,4-hexafluorbutylester mit einem Schmelzpunkt von 84 bis 86°C bei 20 mbar erhalten. Die Reinheit des hergestellten Produkts betrug 95 % (gaschromatographisch bestimmt).

### Beispiel 7

Zu einer Lösung von 55,5 g (0,2 Mol) 2,3-Difluorpropionsäure-2,2,3,4,4,4-hexafluorbutylester und einer Spatelspitze Hydrochinon in 550 ml trockenem Diethylether wurde bei -10°C eine Lösung von 23 g (0,19 Mol) 1,5-Diazabicyclo[4.3.0]non-5-en in 140 ml trockenem Diethylether getropft. Danach wurde das Lösungsmittel in der Kälte im Vakuum abgezogen und der verbleibende Rückstand fraktioniert destilliert. So wurden 23 g α-Fluoracrylsäure-2,2,3,4,4,4-hexafluorbutylester mit einem Siedepunkt von 53 bis 54°C bei 20 mbar in 98 %iger Reinheit erhalten.

## Patentansprüche

1. Verfahren zur Fluorierung von Acrylsäure und Derivaten davon, dadurch gekennzeichnet, daß man an die C=C-Doppelbindung von Acrylsäure oder einem Derivat davon mit der Formel (II) 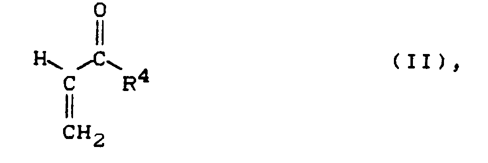 in der
R⁴ für OH, ein Halogenatom oder einen gegebenenfalls substituierten Rest aus der Gruppe C₁- bis C₂₀-Alkoxy, C₃- bis C₂₀-Cycloalkoxy, C₆- bis C₂₀-Aryloxy und C₇- bis C₂₀-Aralkoxy steht
in Gegenwart eines Lösungsmittels elementares Fluor addiert und so 2,3-Difluorpropionsäure oder ein Derivat davon mit der Formel (III) erhält 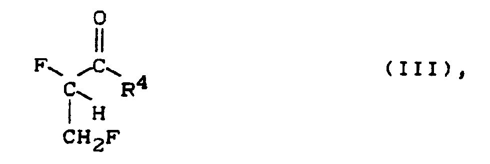 in der
R⁴ die bei Formel (II) angegebene Bedeutung hat.

2. Verfahren nach Anspruch 1, dadurch gekennzeichnet, daß man als Verbindung der Formel (II) Acrylsäurefluorid oder Acrylsäurechlorid einsetzt.

3. Verfahren nach Anspruch 1, dadurch gekennzeichnet, daß man Verbindungen der Formel (II) einsetzt, bei denen R⁴ für einen teilfluorierten aliphatischen C₄- bis C₂₀-Alkoxyrest steht, der geradkettig, verzweigt oder cyclisch sein kann.

4. Verfahren nach Ansprüchen 1 bis 3, dadurch gekennzeichnet, daß man die Umsetzung mit elementarem Fluor bei -100 bis +30°C vornimmt.

5. Verfahren nach Ansprüchen 1 bis 4, dadurch gekennzeichnet, daß man 0,8 bis 1,5 Mol elementares Fluor pro Mol der Verbindung der Formel (II) einsetzt.

6. Verfahren nach Ansprüchen 1 bis 5, dadurch gekennzeichnet, daß man das Fluor als Fluor/Inertgas-Gemisch einsetzt, das 0,5 bis 50 Gew-% elementares Fluor enthält.

7. Verfahren nach Ansprüchen 1 bis 6, dadurch gekennzeichnet, daß man anschließend Verbindungen der Formel (III) durch Abspaltung von Fluorwasserstoff und gegebenenfalls eine Umderivatisierung in α-Fluoracrylsäure oder ein Derivat davon mit der Formel (I) 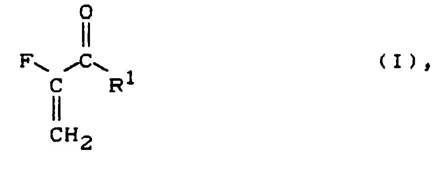 in der
R¹ für NR²R³ oder OR² steht, wobei R² Wasserstoff oder einen gegebenenfalls substituierten Rest aus der Gruppe C₁- bis C₂₀-Alkyl, C₃- bis C₂₀-Cycloalkyl, C₆- bis C₂₀-Aryl und C₇- bis C₂₀- Aralkyl bedeutet und R³ den gleichen Bedeutungsumfang hat wie R² und gleich oder verschieden von R² sein kann,
überführt.

8. Verfahren nach Anspruch 7, dadurch gekennzeichnet, daß man die Abspaltung von Fluorwasserstoff durch Zusatz einer Base durchführt.

9. Verfahren nach Ansprüchen 7 und 8, dadurch gekennzeichnet, daß man die Abspaltung von Fluorwasserstoff bei -78 bis +100°C durchführt.

10. Fluorierte Ester der 2,3-Difluorpropionsäure der Formel (IIIa) 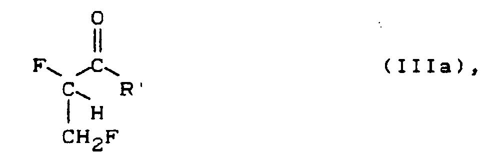 in der
R′ für einen mit Fluor substituierten C₁- bis C₂₀-Alkoxy- oder C₃- bis C₂₀-Cycloalkoxyrest stehen.

## Claims

1. Process for the fluorination of acrylic acid and derivatives thereof, characterized in that elemental fluorine is brought in the presence of a solvent into an addition reaction with the C=C double bond of acrylic acid or of a derivative thereof of the formula (II) 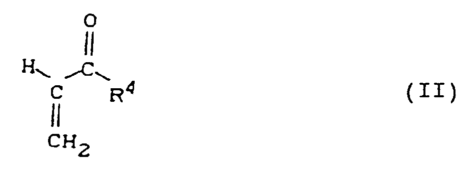 in which
R⁴ represents OH, a halogen atom or a substituted or unsubstituted radical from the group C₁- to C₂₀-alkoxy, C₃-to C₂₀-cycloalkoxy, C₆- to C₂₀-aryloxy and C₇- to C₂₀-aralkoxy
and in this way 2,3-difluoropropionic acid or a derivative thereof having the formula (III) is obtained 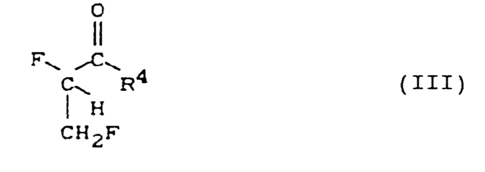 in which
R⁴ has the meaning given in formula (II).

2. Process according to Claim 1, characterized in that the compound used of the formula (II) is acryloyl fluoride or acryloyl chloride.

3. Process according to Claim 1, characterized in that compounds of the formula (II) are used in which R⁴ represents a partially fluorinated aliphatic C₄- to C₂₀-alkoxy radical which may be straight chain, branched or cyclic.

4. Process according to Claims 1 to 3, characterized in that the reaction with elemental fluorine is carried out at -100 to +30°C.

5. Process according to Claims 1 to 4, characterized in that 0.8 to 1.5 moles of elemental fluorine are used per mole of compound of the formula (II).

6. Process according to Claims 1 to 5, characterized in that the fluorine is employed in the form of a fluorine/inert gas mixture which contains 0.5 to 50% by weight of elemental fluorine.

7. Process according to Claims 1 to 6, characterized in that compounds of the formula (III) are subsequently converted by elimination of hydrogen fluoride and, if desired, by forming another derivative into a-fluoroacrylic acid or a derivative thereof of the formula (I) 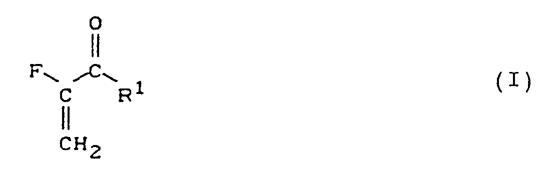 in which
R¹ represents NR²R³ or OR², R² being hydrogen or a substituted or unsubstituted radical from the group C₁- to C₂₀-alkyl, C₃- to C₂₀-cycloalkyl, C₆- to C₂₀-aryl and C₇- to C₂₀-aralkyl and R³ has the same scope of meaning as R² and may be identical with or different from R².

8. Process according to Claim 7, characterized in that the elimination of hydrogen fluoride is carried out by adding a base.

9. Process according to Claims 7 and 8, characterized in that the elimination of hydrogen fluoride is carried out at -78 to +100°C.

10. Fluorinated esters of 2,3-difluoropropionic acid of the formula (IIIa) 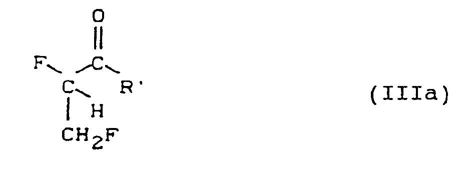 in which
R¹ represents a fluorine-substituted C₁- to C₂₀ alkoxy radical or C₃- to C₂₀-cycloalkoxy radical.

## Revendications

1. Procédé de fluoration d'acide acrylique et de ses dérivés, caractérisé en ce qu'on additionne du fluor élémentaire sur la double liaison C=C de l'acide acrylique ou d'un dérivé de cet acide de formule (II) 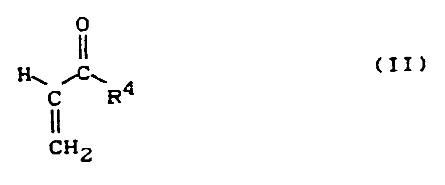 dans laquelle
R⁴ représente OH, un atome d'halogène ou un reste éventuellement substitué du groupe des restes alkoxy en C₁ à C₂₀, cycloalkoxy en C₃ à C₂₀, aryloxy en C₆ à C₂₀ et aralkoxy en C₇ à C₂₀
en présence d'un solvant, et on obtient ainsi l'acide 2,3-difluoropropionique ou un dérivé de cet acide de formule (III) 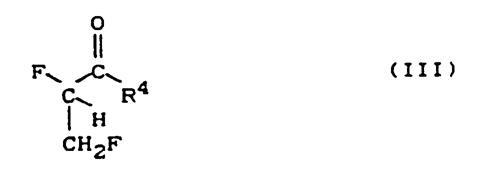 dans laquelle R⁴ a la définition indiquée pour la formule (II).

2. Procédé suivant la revendication 1, caractérisé en ce qu'on utilise comme composé de formule (II) le fluorure d'acide acrylique ou le chlorure d'acide acrylique.

3. Procédé suivant la revendication 1, caractérisé en ce qu'on utilise des composés de formule (II) dans lesquels R⁴ représente un reste alkoxy aliphatique en C₄ à C₂₀ partiellement fluoré qui peut être à chaîne droite, à chaîne ramifiée ou cyclique.

4. Procédé suivant les revendications 1 à 3, caractérisé en ce qu'on conduit la réaction avec du fluor élémentaire entre -100 et +30°C.

5. Procédé suivant les revendications 1 à 4, caractérisé en ce qu'on utilise 0,8 à 1,5 mole de fluor élémentaire par mole de composé de formule (II).

6. Procédé suivant les revendications 1 à 5, caractérisé en ce qu'on utilise le fluor sous forme d'un mélange de fluor et de gaz inerte qui contient 0,5 à 50 % en poids de fluor élémentaire.

7. Procédé suivant les revendications 1 à 6, caractérisé en ce qu'on transforme ensuite des composés de formule (III) par élimination de fluorure d'hydrogène et, le cas échéant, par une re-dérivatisation, en acide α-fluoracrylique ou un dérivé de cet acide de formule (I) 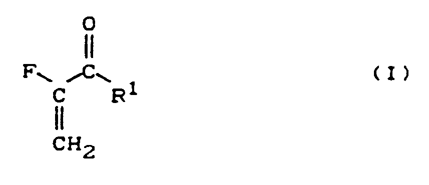 dans laquelle
R¹ est un groupe NR²R³ ou OR², R² représentant l'hydrogène ou un reste éventuellement substitué du groupe des restes alkyle en C₁ à C₂₀, cycloalkyle en C₃ à C₂₀, aryle en C₆ à C₂₀ et aralkyle en C₇ à C₂₀, et R³ a la même définition que R² et peut être identique à R² ou peut en être différent.

8. Procédé suivant la revendication 7, caractérisé en ce qu'on conduit l'élimination de fluorure d'hydrogène par addition d'une base.

9. Procédé suivant les revendications 7 et 8, caractérisé en ce qu'on conduit l'élimination du fluorure d'hydrogène entre -78 et +100°C.

10. Esters fluorés de l'acide 2,3-difluoropropionique de formule (IIIa) 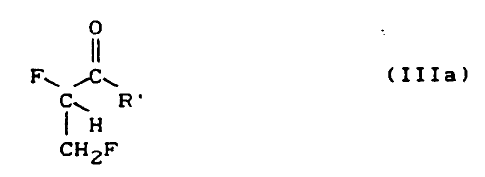 dans laquelle
R′ est un reste alkoxy en C₁ à C₂₀ ou cycloalkoxy en C₃ à C₂₀ substitué avec du fluor.
